# EUROPEAN PATENT APPLICATION

(11) **EP 2 572 662 A1**
(43) Date of publication of application: **27.03.2013**
(21) Application number: 11306209.5
(22) Date of filing: 23.09.2011
(51) Int. Cl.: A61B 17/70

(54) **Stabilization device for vertebrae**

(71) Applicant: ZIMMER SPINE, 33000 Bordeaux (FR)
(72) Inventor: Larroque-Lahitette, Gilles, 33750 BARON (FR); Belliard, Karl, 49770 LA MEMBROLLE SUR LONGUENEE (FR)
(74) Representative: Besnard, Christophe Laurent

(57) **Abstract**

Device for stabilizing at least two vertebrae (V1, V2), comprising a plate (10) adapted to be fixed to the vertebrae (V1, V2), along vertebral bodies (VB1, VB2) of the vertebrae (V1, V2). The plate (10) has caudal (10Cd), cranial (10Cr) and lateral sides (10S), and at least one pair of two arms (2, 3) joined to the plate, and essentially oppositely extending from the lateral sides (10S) of the plate. The two arms (2, 3) are configured to embrace the vertebral body of a vertebra and/or a vertebral body replacement. Method for stabilizing at least two vertebrae (V1, V2) and a possible vertebral body replacement, using such a device.

## Description

### FIELD OF THE INVENTION

The present disclosure relates to a prosthetic device and method for stabilizing at least two vertebrae.

### BACKGROUND

One field of application for the invention is stabilizing vertebrae, i.e. holding together at least two vertebrae in a desired configuration, for example to aid in healing of spinal traumas or to correct spinal structure deficiencies and abnormalities.

The spine is formed of superposed vertebrae, normally aligned along the middorsal line, from the lumbar vertebrae to the cervical vertebrae, each having an anterior part: the vertebral body, and a posterior part: the vertebral arch (or neural arch), the anterior and posterior parts enclosing the vertebral foramen. Each vertebral arch is formed by a pair of pedicles (joined to the vertebral body) and a pair of laminae, with articular processes transverse processes and/or one spinous process (or neural spine) projecting therefrom. The transverse processes and the spinous process project opposite to the vertebral foramen. When the vertebrae are articulated with each other, the vertebral bodies form a strong pillar for the support of the head and trunk. In between every pair of vertebral bodies, there is an intervertebral disc.

Damage to the spine resulting from disease, the effects of aging or physical trauma, is treated in many instances by stabilization of vertebrae. The use of plates and screws for stabilization of the vertebrae has been widely accepted as a reliable practice and has proven to be successful clinically. Such plates are attached to the vertebral bodies, i.e. to the anterior part of the vertebrae, through an anterior (or antero-lateral) approach, i.e. via the front of a patient. Compared to a posterior approach (i.e. via the back of the patient), anterior approach techniques provide the widest access to the vertebrae.

Conventional plates are attached to the vertebrae by bone screws which are screwed into the vertebral bodies of the vertebrae. However, in some cases, the vertebral bodies do not provide sufficient purchase (e.g. in case of poor bone quality) to fixedly and permanently hold the screws, which may lead to a weak connection between the plate and the vertebrae and, consequently, to a poor or void stabilization effect.

Accordingly, there is a need for an improved prosthetic device and method for stabilizing vertebrae.

### SUMMARY OF THE INVENTION

According to one aspect of the present disclosure, there is provided a device for stabilizing at least two vertebrae, the device comprising:
a plate adapted to be fixed to the vertebrae, along the vertebral bodies of the vertebrae, the plate having caudal, cranial and lateral sides, and
at least one pair of two arms joined to the plate, and essentially oppositely extending from the lateral sides of the plate.

In particular, the two arms of said pair(s) are configured to embrace (i.e. to closely surround, partially or completely) a vertebral body of a vertebra and/or a vertebral body replacement.

A vertebral body replacement, abbreviated to "VBR" ("VBRs" in plural form), is a prosthetic structure used to replace a weakened vertebra, once removed. Examples of VBRs include structural bone struts made from autograft or allograft tissue, biocompatible porous structures (e.g. tantalum biocompatible composition), vertebral cages (e.g. structural titanium mesh cages), etc. VBRs may be expandable along the middorsal line. VBRs are mainly implanted using an anterior approach which allows for greater access to the vertebrae.

Such a stabilization device may be provided with one or several pairs of arms, the two arms of a pair extending from the opposite lateral sides of the plate, respectively, and being configured to embrace the vertebral body of a vertebra or the VBR. Each arm has a proximal end joined to the plate and a distal end. The proximal ends extend from the lateral sides of the plate, in opposite directions. The distal ends of the two arms may be joined to each other, thereby defining with the plate a ring, or they may be free, thereby defining with the plate a "C" shape. In general, when the two arms embrace a vertebral body, their proximal ends are free since, in practice, it is difficult for the physician (or other operative) to completely surround (i.e. to encircle) the vertebra with the arms because of the spinal cord located on the posterior side of the vertebral body. When the two arms embrace a VBR, their proximal ends may be joined to each other or be free (i.e. not joined), so as to surround partially or completely the VBR.

Such a stabilization device may be used for providing "static stabilization" or "dynamic stabilization" between the vertebrae. Static stabilization consists in holding together the vertebrae in a particular relative position, while not allowing any movement between the vertebrae, whereas dynamic stabilization consists in holding together the vertebrae in a particular relative position, while allowing a limited amount of relative movement between the vertebrae. For dynamic stabilization, the arms may have elastic properties.

Compared to conventional screws, the stabilization device provides a better connection between the plate and the vertebrae by decreasing the stress on the anatomy and providing a good connection while being less dependent on the bone quality of the vertebral body. Moreover, said arms may fit on any vertebral body geometry, or VBR geometry.

In certain embodiments, said two arms are made by at least one flexible elongate member. In particular, the two arms may be made either by one flexible elongate member, or respectively by two flexible elongate members. In other terms, one flexible elongate member forms the two arms, or each arm is formed by a separate flexible elongate member.

Said flexible elongate member(s) may be passed around a bony structure of the vertebra, typically a transverse process, or around said VBR. In each case, said flexible elongate member or members closely surround the vertebral body of the vertebra or the VBR.

The flexible elongate member may be any tie that is flexible such as a band, a wire, a ribbon, a strap or a cord made of metal, polymeric material or a combination thereof. The elongate member may be made from a polymeric material such as polyester, polyethylene (for example, polyethylene terephthalate or PET), polyetheretherketone (PEEK) or any other material that provides the desired strength and flexibility. When the flexible elongate member is band-shaped (i.e. the elongate member is a thin and wide band of material), the interface between the elongate member and the anatomy (or the VBR) is wide, thereby improving the contact and better distributing the stresses on the anatomy (or the VBR), thus avoiding cutting phenomenon.

In certain embodiments, the flexible elongate member defines a loop configured to surround a bony structure of the vertebra (generally a transverse process) or said VBR.

In certain embodiments, the flexible elongate member defines two loops, one on each side of the plate, each loop being configured to surround a transverse process of the vertebra.

So, when two loops extend respectively from the opposite lateral sides of the plate, each loop may be defined by one elongate member, or the two loops may be defined by the same elongate member which, in this latter case, may be bent so as to form a "8" or equivalent.

In certain embodiments, the plate comprises one or several passages through which the elongate member passes. Such passages allow the elongate member(s) to be guided with respect to the plate, properly tensioned and/or joined to the plate. These passages may extend through the thickness of the plate or through protrusions provided on the anterior face of the plate, for example.

In certain embodiments, the two arms constitute a clamping device. In particular, the two arms may be configured to clamp the vertebral body or the VBR between them. In this case, the two arms may form clamping jaws adapted to exert clamping strain on the vertebral body or the VBR located therebetween.

In certain embodiments, at least one of the two arms is an elastic member and is, in particular, configured such that the two arms exert a clamping force on the vertebral body or the VBR, when mounted thereon.

In certain embodiments, the stabilization device comprises a locking system for urging said arms towards each other in order to exert a clamping force on the vertebral body or the VBR.

According to another aspect of the present disclosure, there is provided a set comprising a stabilization device as described above and a vertebral body replacement (abbreviated to "VBR"). In this case, the stabilization device is fixed to the vertebrae surrounding the VBR, and to the VBR, thereby providing stabilization between the vertebrae while holding the VBR in position.

According to another aspect of the present disclosure, there is provided a method for stabilizing at least two vertebrae, the method comprising the steps of:
providing a stabilization device as described above, and fixing the plate to the vertebrae, along the vertebral bodies of the vertebrae, wherein the plate is fixed to at least one vertebra by embracing the vertebral body of this vertebra in said arms.

Depending on the length of the plate along the middorsal line, more than two vertebrae may be stabilized. Moreover, depending on the number of fixation systems for fixing the plate on the vertebrae, the plate may be fixed to more than two vertebrae. At least one of the fixation systems or all the fixation systems may be of the above-mentioned type, with two embracing arms.

According to another aspect of the present disclosure, there is provided a method for stabilizing at least two vertebrae and a vertebral body replacement (abbreviated to "VBR") located between these two vertebrae, the method comprising the steps of:
providing a VBR,
placing the VBR between two vertebrae,
providing a stabilization device as described above,
fixing the plate on the VBR, and
fixing the plate to the vertebrae, along the vertebral bodies of the vertebrae, wherein the plate is fixed to at least one vertebra and/or to the VBR by embracing the vertebral body of this vertebra or the VBR in said arms.

In this case, the plate is provided with at least three fixation systems: two for fixing the plate to the vertebrae, and one for fixing the plate to the VBR. At least one of these fixations systems or all the fixation systems may be of the above-mentioned type, with two embracing arms.

In both methods, the plate is attached along an anterior surface of the vertebrae through an anterior (or antero-lateral) approach.

In both methods, the fixation systems used for fixing the plate on the vertebrae (or the VBR) may be analogous or different from each other. In particular, either all the fixation systems are of the above-mentioned type, with two embracing arms, or only some of them are, the other(s) being conventional fixation systems such as bone screws or any other type of anchors.

Other features and advantages of the invention will become apparent from the following detailed description, taken in conjunction with the accompanying drawings which illustrate, by way of example, the principles of the invention.

### BRIEF DESCRIPTION OF THE DRAWINGS

In the drawings, like reference signs generally refer to the same parts throughout the different views. Moreover, parts belonging to different embodiments but having analogous functions are given like reference numbers identified by the same reference numerals spaced from 100, 200, etc.

The drawings are not necessarily to scale, emphasis instead being placed upon illustrating the principles of the invention.
FIG 1 is an anterior view of an example of a stabilization device mounted on two adjacent vertebrae.
FIG 2 is an anterior view, analogous to FIG 1, showing another example of a stabilization device.
FIG 3 is a sectional view of the device of FIG 2, along the transversal plane III-III.
FIG 4 is an anterior view, analogous to FIG 1, showing another example of a stabilization device.
FIG 5 is a perspective view showing in detail one fixation system of the device of FIG 4.
FIG 6 is a sectional view of the device of FIG 4, along the transversal plane VI-VI.
FIG 7 is a sectional view of the device of FIG 4, along the transversal plane VII-VII.
FIG 8 is an anterior view of an example of a set comprising a vertebral body replacement (or VBR) and a stabilization device, the stabilization device being fixed to the VBR and mounted on the vertebrae adjacent to the VBR.
FIG 9 is a side view of the example of FIG 8.

### DETAILED DESCRIPTION

In the following detailed description, it is referred to the accompanying drawings showing examples of stabilization devices. It is intended that these examples be considered as illustrative only, the scope of the invention not being limited to these examples.

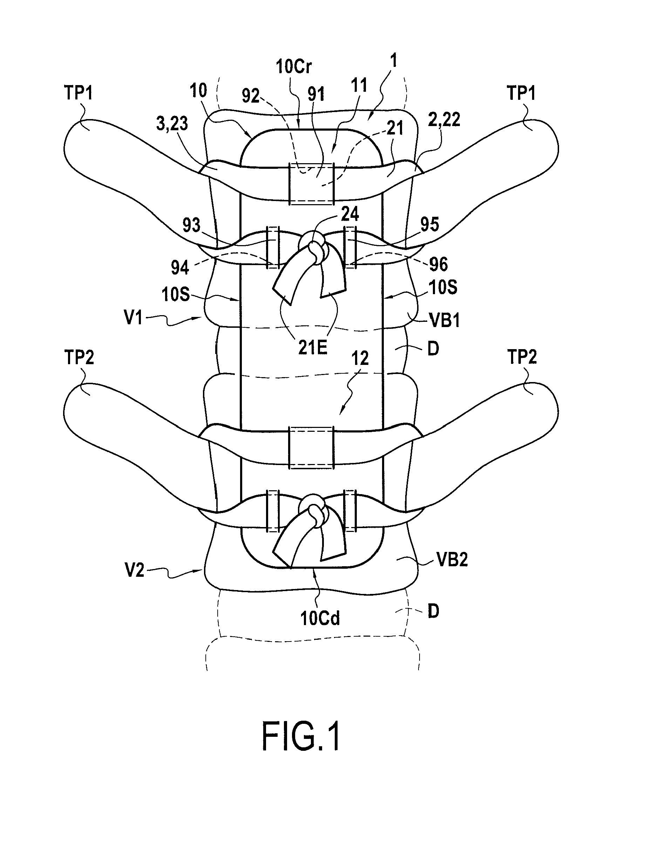

A first example of stabilization device 1 is shown in FIG 1. The device 1 comprises a thin elongate plate 10 to be mounted on two superposed vertebrae V1, V2. More precisely, the plate is adapted to rest on the anterior surfaces of the vertebral bodies VB1, VB2 of the vertebrae V1, V2, as shown in FIG 1. The longitudinal dimension of the plate 10 is oriented substantially along the middorsal line. The plate 10 has a cranial side 10Cr, a caudal side 10Cd and two opposite lateral sides 10S. When the plate 10 is implanted on the vertebrae V1, V2, the cranial side 10Cr is oriented towards the crane of the patient and the caudal side 10Cd is oriented towards the "tail" of patient's spine. The plate may be dimension such that the width (i.e. the lateral dimension) of the plate 10 is smaller than the width of the vertebral bodies VB1, VB2.

The plate 10 is provided with two fixation systems for fixing the plate 10 to the vertebrae V1, V2. One fixation system 12 is at the height of the lower vertebra V2, and the other fixation system 11 is at the height of the upper vertebra V1. The vertebrae V1, V2 are separated by an intervertebral disc D.

In this example the upper and lower fixation systems 11, 12 are similar (of course, they might be different) and, therefore, only the upper fixation system 11 is described hereunder.

The fixation system 11 comprises a flexible elongate member which is, in this example, a band-shaped tie 21 made of polymeric material. The fixation system 11 further comprises three passages 92, 94, 96 through which the tie 21 can be passed. These three passages 92, 94, 96 are shown in dotted lines in FIG 1. The first passage 92 is made through a protruding part 91 (e.g. a bulging part) on the anterior face of the plate 10. Passage 92 extends along the transversal direction of the plate 10, i.e. along the left-right direction when the plate 10 is in place on the vertebrae as shown in FIG 1. Thus, the protruding part 91 and the first passage 92 form a kind of belt ring through which the tie 21 can be passed. The second and third passages 94, 96 are located below the first passage 92. They are also made through protruding parts 93, 95 on the anterior face of the plate 10, and they extend along the transversal direction of the plate 10, i.e. along the left-right direction. The second and third passages 94, 96 are at the same height (with respect to the vertical direction when the plate 10 is in place on the vertebrae, as shown in FIG 1) but are separated by a space in the left-right direction.

The tie 21 has two end portions 21E. The tie 21 is first passed through the first passage 92, such that end portions 21E extend, respectively, from the opposite lateral sides 10S of the plate 10. Then, each end portion 21E is passed around a transverse process TP1 of the vertebra V1. It is to be noted that end portions 21E could be passed around other bony structures of the vertebra V1, such as the pedicles, if desired. Then, the end portions 21E are passed through the second and third passages 94, 96, respectively. Thus, the tie 21 forms two loops 22, 23, extending from the opposite lateral sides 10S of the plate 10, each loop 22, 23 surrounding one transverse process TP1, or other bony structure, of the vertebra V1. The loops 22, 23 constitute arms 2, 3, in the sense of the present disclosure.

For fixing the plate 10 to the vertebra V1, the end portions 21E are pulled so as to tension the tie 21. Thus, the loops 22, 23 are tightened around the transverse processes TP1, or other bony structure. Then, the end portions 21E are held in position with respect to the plate 10.

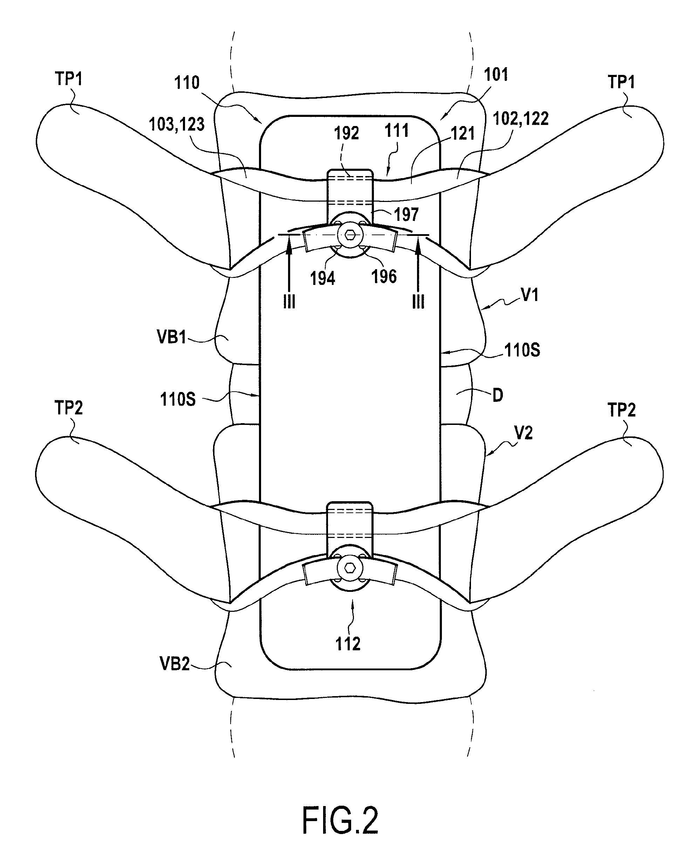

For holding the end portions 21E in position with respect to the plate 10, the end portions 21E may be knotted (knot 24), as shown in FIG 1. Alternatively, a locking mechanism may be used to secure the end portions 21E in position. An example of a locking mechanism is described below, with reference to FIGS 2-3. Of course, other kinds of locking mechanisms might be used.

In the example of FIG 2, the plate 110 is provided with two fixation systems for fixing the pate 110 to the vertebrae V1, V2. One fixation system 112 is at the height of the lower vertebra V2, and the other fixation system 111 is at the height of the upper vertebra V1. The vertebrae V1, V2 are separated by an intervertebral disc D.

In this example the upper and lower fixation systems 111, 112 are similar (of course, they might be different) and, therefore, only the upper fixation system 111 is described hereunder.

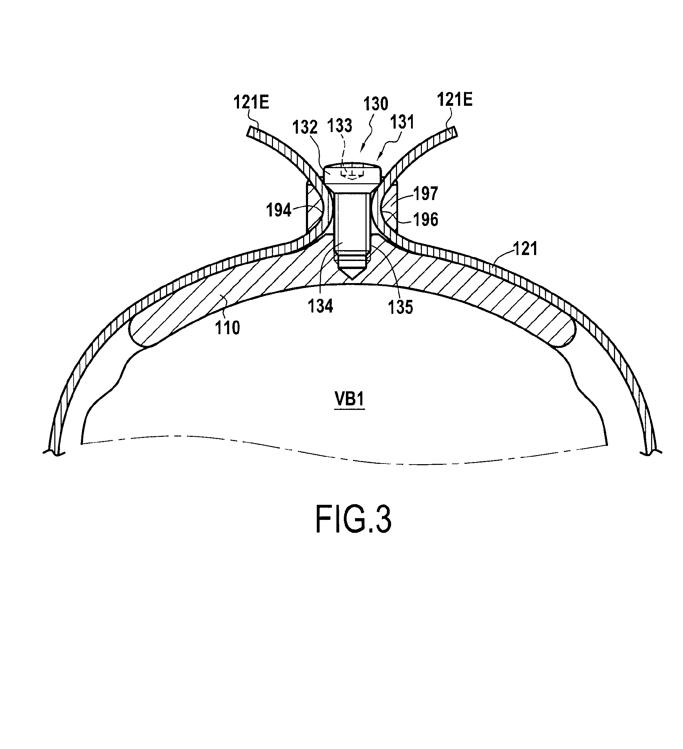

The fixation system 111 comprises a flexible elongate member which is, in this example, a band-shaped tie 121 made of polymeric material. The fixation system 111 further comprises three passages 192, 194, 196 through which the tie 121 can be passed. The first passage 192 is shown in dotted lines in FIG 2. Passage 192 is made through a protruding part 197 (e.g. a bulging part) on the anterior face of the plate 110. Passage 192 extends along the transversal direction of the plate 110, i.e. along the left-right direction when the plate 110 is in place on the vertebrae as shown in FIG 2. Thus, the protruding part 197 and the first passage 192 form a kind of belt ring through which the tie 121 can be passed. The second and third passages 194, 196 are located below the first passage 192. They are also made through the protruding part 197 as shown in FIG 3. Passages 194, 196 are "C" shaped, extending first along the transversal direction of the plate 110, and then perpendicular to the plate 110. Second and third passages 194, 196 are at the same height (with respect to middorsal direction, as shown in FIG 2) but are spaced out in the left-right direction. In the space between the second and third passages 194, 196, there is a threaded hole 135 extending through the thickness of the plate 110 in the antero-posterior direction.

The fixation system 111 further comprises a locking mechanism 130 with a screw 131 having a head 132 and a shank 134 with an external thread, the external thread of the shank 134 engaging with the threaded hole 135 in the plate 110. The screw head 132 has a profile 133 (a hollow hexagonal profile in this example) that allows the screw 131 to be driven in rotation.

The second and third passages 194, 196 are provided on each side of the threaded hole 135 and the lower face of the screw head 132 is conical and large enough to extend over the exit opening of the passages 194, 196. As a consequence, the tie end portions 121E (see FIG 3) passing through the passages 194, 196, pass under the screw head 132 and, when the screw 131 is screwed into the plate 110, the tie 121 is locked in position by being compressed between the screw head 132 and the protruding part 197.

Like in FIG 1, the loops 122, 123 defined by the tie 121 on each side of the plate 110 constitute the arms 102, 103 of the stabilization device 101.

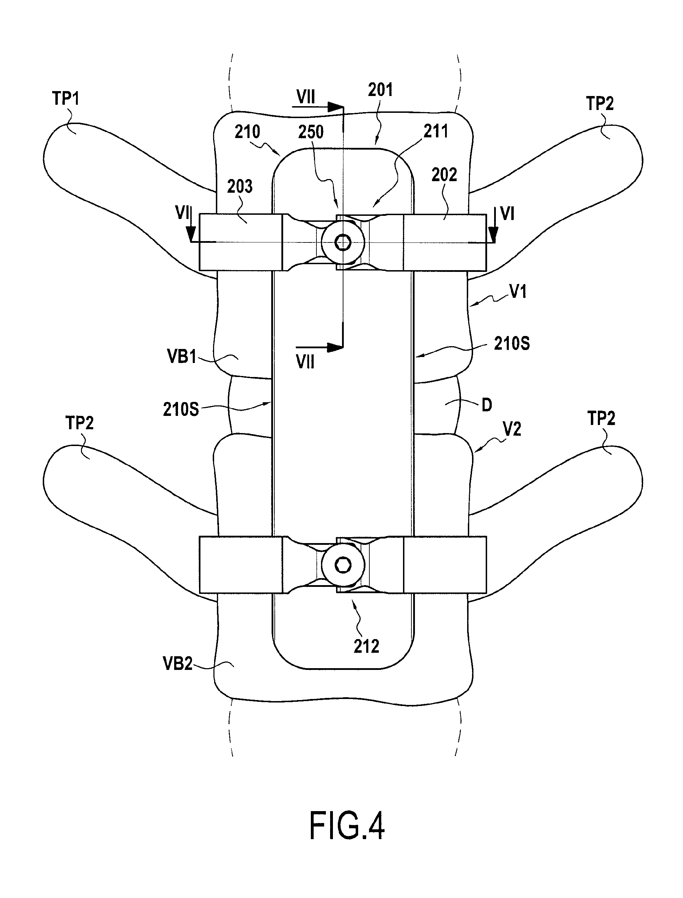

Another example of a stabilization device 201 is shown in FIGS 4-7. The device 201 comprises a plate 210 and two fixation systems 211, 212 for fixing the pate 210 to the vertebrae V1, V2. These two fixation systems 211, 212 are similar and, therefore, only the upper fixation system 211 is described hereunder. The fixation systems 211, 212 differ from the ones of examples of FIGS 1-3 in that the arms 202, 203 extending from the opposite lateral sides 210S of the plate 210 are rigid members acting as clamping jaws, instead of being made by a flexible tie. The stabilization device 201 further comprises a locking system 250 for urging the arms 202, 203 towards each other so as to exert a clamping force on the vertebral body VB1 (or VB2).

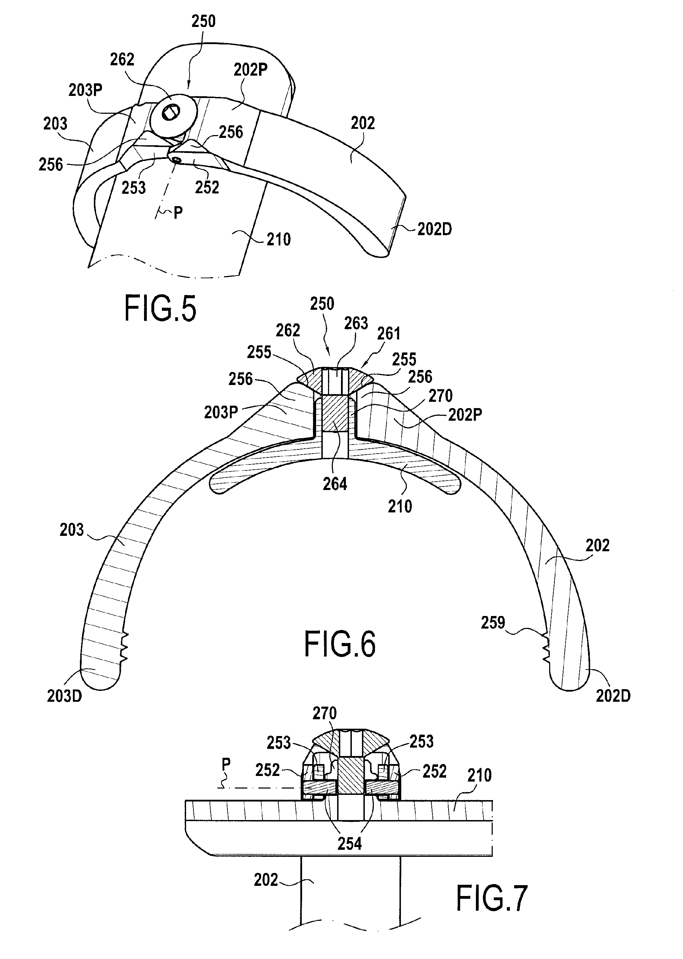

In this example, the anterior face of the plate 210 bears an upstanding tube 270 (see FIG 6) provided with an internal thread. The locking system 250 comprises a screw 261 having a head 262 and a shank 264 with an external thread, the external thread of the shank 264 engaging with the internal thread of the upstanding tube 270. The screw head 262 has a profile 263 (e.g. a hollow hexagonal profile - see FIG 5) that allows the screw 261 to be driven in rotation. The lower face of the screw head 262 is conical.

The two arms 202, 203 are hinged together at their proximal ends 202P, 203P, or are otherwise pivotable with respect to each other. More precisely, the proximal end 202P of the arm 202 is bracket shaped, with a pair of first fingers 252 spaced out from each other. In other terms, the proximal end 202P has substantially the shape of a two-teeth fork. The proximal end 203P of the arm 203 is also bracket shaped, with a pair of second fingers 253 spaced out from each other. The space between the second fingers 253 is smaller that the space between the first fingers 252, so that second fingers 253 may be inserted between the first fingers 252, each first finger 252 being close to one second finger 253. The space between the second fingers 253 is wide enough to receive the upstanding tube 270. The fingers 252 and 253 are hinged together and with the upstanding tube 270, by means of two pins 254 being diametrically opposed with respect to the center line of the upstanding tube 270. Each pin 254 goes through one first finger 252, one second finger 253 and the upstanding tube 270. As a result, the two arms 202, 203 are adapted to pivot with respect to the upstanding tube 270. It is noted that in other instances, the fingers of the two arms 202, 203 may be otherwise arranged to permit pivotable movement therebetween.

Each proximal end 202P, 203P is further provided with a protrusion 256 on its outer face, the protrusion 256 defining a slopped surface 255. The slopped surfaces 255 define together a V-shaped profile, as shown in FIG 6. When the screw 261 is screwed into the tube 270, the lower face of the screw head 262 pushes on the slopped faces 255, so as to make the arms 202, 203 pivot around the axis P of the pins 254. As a result, the arms 202, 203 move from an open (or unclamped) configuration to a close (or clamped) configuration.

When a vertebral body (or a VBR) is located between the arms 202, 203, the arms are urged towards the vertebral body (or the VBR), thereby clamping the vertebral body (or the VBR) between the arms 202, 203. In order to improve the contact between the arms and the vertebral body (or the VBR), the distal ends 202D, 203D of the arms 202, 203, may be provided with protrusions such as spikes 259, on their inner faces (see FIG 6) to engage the vertebral body (or the VBR).

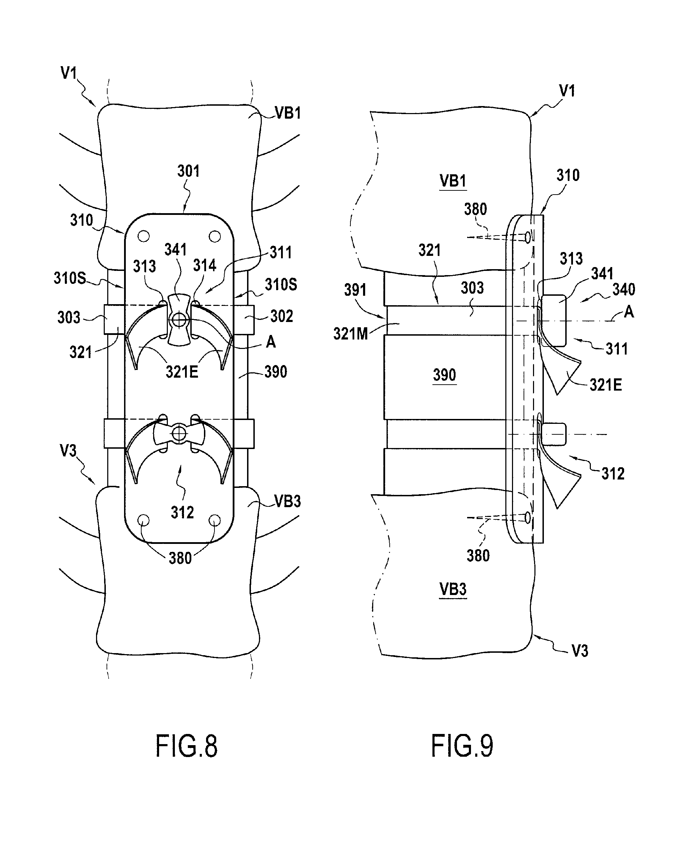

Another example of a stabilization device 301 is shown in FIGS 8-9. This device 301 is comprised in a set further comprising a vertebral body replacement 390, abbreviated to "VBR". The VBR 390 may be a biocompatible porous structure, or other load bearing structure for positioning between vertebrae. For instance, a VBR made from biocompatible porous tantalum composition, such as the one marketed by ZIMMER SPINE under the name "Trabecular Metal VBR", may be used.

In the example of FIGS 8-9, the vertebra V2 between vertebrae V1 and V3 has been removed and replaced by the VBR 390.

The device 301 comprises a plate 310 which is fixed to the vertebral body VB1 of vertebra V1 and to the vertebral body VB2 of vertebra V2 by means of regular bone screws 380. Of course, plate 310 could be fixed to vertebrae V1 and/or V2 by means of arms embracing the vertebral body VB1 and/or VB2, such as the arms 2, 3, 102, 103, 202, 203 shown in FIGS 1-7 (i.e. by means of flexible elongate members like in FIGS 1-3, or clamping arms like in FIGS 4-7).

The plate 310 is further provided with two fixation systems 311, 312 for fixing the pate 310 to the VBR 390. Fixation systems 311, 312 are spaced out along the longitudinal direction of plate 310, i.e. along the vertical direction as shown in FIG 8. In this example the upper and lower fixation systems 311, 312 are similar (of course, they might be different) and, therefore, only the upper fixation system 311 is described hereunder.

Fixation system 311 comprises first and second arms 302, 303 joined to the plate 310. The arms 302, 303 of one pair extend from opposite lateral sides 310S of the plate 310, respectively, and are configured to embrace the VBR 390.

In this example, each pair of arms 302, 303 is made by one flexible elongate member or tie 321. The tie 321 has two end portions 321E and an intermediate portion 321M therebetween. Intermediate portion 321M surrounds the VBR 390 while the two end portions 321E pass, respectively, through two passages provided in the plate 310. In this example, these passages are simple slots 313, 314 extending through the thickness of the plate 310. The plate 310 is further provided with a locking mechanism 340 comprising a compression member 341 which is rotatably mounted on the plate 310, the compression member 341 being rotatable around an axis A perpendicular to the plate 310 (see FIGS 8-9). The slots 313, 314 are provided on each side of axis A. The compression member 341 is not axisymmetric around axis A: in this example, compression member 341 is bow-tie shaped. The compression member 341 is rotatable between a first, or open, position, where the compression member 341 does not extend over the slots 313, 314, and a second, or close, position where the compression member 341 extends over the slots 313, 314. In FIG 8, the compression member 341 of the upper fixation system 311 is shown in the open position, while the compression member 341 of the lower fixation system 312 is shown in the close position.

The tie 321 is passed around the VBR 390 and the end portions 321E are passed through the slots 313, 314, when the compression member 341 is in the open position. The tie 321 is then tightened around the VBR 390 by pulling on the end portions 321E. The tie 321 is locked in position relative to the plate 310 by turning the compression member 341 around axis A into its close position so as to compress the tie 321 between the compression member 341 and the plate 310.

In order to guide the tie 321 around the VBR 390, the VBR 390 is provided with a circumferential groove 391 (see FIG 9) adapted for receiving the tie 321.

The above description is given by way of example, and not limitation. Given the above disclosure, one skilled in the art could devise variations that are within the scope of the invention disclosed herein. Further, the various features of the embodiments or examples disclosed herein can be used alone or in varying combinations with each other, and are not intended to be limited to the specific combination described herein.

## Claims

1. A device for stabilizing at least two vertebrae, the device comprising:
a plate (10; 110; 210; 310) adapted to be fixed to the vertebrae (V1, V2) along vertebral bodies (VB1, VB2) of the vertebrae (V1, V2), the plate (10; 110; 210; 310) having caudal, cranial and lateral sides, and at least one pair of two arms (2, 3; 102, 103; 202, 203; 302, 303) joined to the plate, and essentially oppositely extending from the lateral sides (10S; 110S; 210S; 310S) of the plate.

2. The device of claim 1, wherein the arms are configured to embrace a vertebral body (VB1, VB2) of a vertebra and/or a vertebral body replacement (390).

3. The device of claim 1 or 2, wherein the two arms (2, 3; 102, 103; 302, 303) are made by at least one flexible elongate member.

4. The device of claim 3, wherein the flexible elongate member defines a loop configured to surround a bony structure of the vertebra (V1, V2), such as a transverse process, or a vertebral body replacement (390).

5. The device of claim 4, wherein the flexible elongate member defines two loops, one on each side of the plate, each loop being configured to surround a transverse process (TP1, TP2) of the vertebra (V1, V2).

6. The device of any one of claims 3 to 5, wherein the plate (10; 110; 310) comprises one or several passages (92, 94, 96; 192, 194, 196; 313, 314) through which the elongate member passes.

7. The device of claim 1 or 2, wherein the two arms (202, 203) constitute a clamping device, the two arms being, in particular, configured to clamp a vertebral body (VB1, VB2) or a vertebral body replacement (390) between them.

8. The device of claim 7, wherein at least one of the two arms is an elastic member and is, in particular, configured such that the two arms exert a clamping force on the vertebral body (VB1, VB2) or the vertebral body replacement (390) when mounted thereon.

9. The device of claim 7 or 8, comprising a locking system (250) for urging the arms (202, 203) towards each other in order to exert a clamping force on the vertebral body (VB1, VB2) or the vertebral body replacement (390).

10. A set comprising a device according to any one of the preceding claims and a vertebral body replacement (390).

11. A method for stabilizing at least two vertebrae (V1, V2), the method comprising:
providing a device (1, 101, 201) according to any one of the preceding claims, and
fixing the plate (10, 110, 210) to the vertebrae (V1, V2), along vertebral bodies (VB1, VB2) of the vertebrae (V1, V2),
wherein the plate (10, 110, 210) is fixed to at least one of the vertebra (V1, V2) by embracing the vertebral body (VB1, VB2) of this vertebra in said arms (2, 3; 102, 103; 202, 203).

12. A method for stabilizing at least two vertebrae (V1, V2) and a vertebral body replacement (390) located between these two vertebrae, the method comprising:
providing a vertebral body replacement (390);
placing the vertebral body replacement (390) between two vertebrae;
providing a device (1, 101, 201, 301) according to any one of the preceding claims,
fixing the plate (10, 110, 210, 310) to the vertebral body replacement (390), and
fixing the plate (10, 110, 210, 310) to the vertebrae (V1, V2), along vertebral bodies (VB1, VB2) of the vertebrae (V1, V2),
wherein the plate (10, 110, 210, 310) is fixed to at least one vertebra (V1, V2) and/or to the vertebral body replacement (390) by embracing the vertebral body (VB1, VB2) of this vertebra and/or the vertebral body replacement (390) in said arms (2, 3; 102, 103; 202, 203; 302, 303).
